# EUROPÄISCHE PATENTSCHRIFT

(11) **EP 2 170 235 B1**
(45) Veröffentlichungstag und Bekanntmachung des Hinweises auf die Patenterteilung: **03.04.2013**
(21) Anmeldenummer: 08758778.8
(22) Anmeldetag: 27.05.2008
(51) Int. Cl.: A61F 13/02, C09J 7/02, C09J 133/00

(54) **MODIFIZIERTE ACRYLATKLEBEMASSEN**
MODIFIED ACRYLATE ADHESIVE MASSES
MATIÈRES ADHÉSIVES ACRYLATES MODIFIÉES

(30) Priorität: 13.07.2007 DE 102007033807
(43) Veröffentlichungstag der Anmeldung: 07.04.2010
(73) Patentinhaber: Beiersdorf AG, 20253 Hamburg (DE)
(72) Erfinder: MEYER-INGOLD, Wolfgang, 22457 Hamburg (DE); ACHTERBERG, Volker, 22159 Hamburg (DE)
(74) Vertreter: Hartmann, Jost
(86) Internationale Anmeldenummer: PCT/EP2008/004192
(87) Internationale Veröffentlichungsnummer: WO 2009/010120

(56) Entgegenhaltungen:
- EP-A- 0 554 106
- WO-A-91/16387
- US-A- 4 554 324
- US-A- 4 737 410
- US-A1- 2003 221 776

## Beschreibung

Die Erfindung beschreibt Acrylatkebemassen, die durch Zusatz von mit Acrylsäure und/oder substituierter Acrylsäure veresterten Wollwachsalkoholen, Fettalkoholen und/oder Sterolen die Haftung, Enthaftung und Hautverträglichkeit der Klebemassen verbessern.

Bekannt sind Pflasterklebemassen auf Basis von Kautschuk (seit 1870), Kautschuk-Zinkoxid (Leukoplast 1901) sowie thermoplastische Heißschmelzklebemassen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich.

In der DE 1719167 wird eine Oberflächenschutzfolie beschrieben, die mit einer Beschichtung aus Acrylkautschuk, keiner reinen Acrylatmasse, versehen ist.
In den Acrylsäureester sind als Akoholkomponente Alkylalkohole mit nicht mehr als 12 Kohlenstoffatomen enthalten. Um ein vernünftiges Abrollen der Oberflächenschutzfolie von der Rolle zu erzielen, wird nicht eine leichte Ablösbarkeit durch die Klebemasse angestrebt, sondern das leichte Abrollen wird durch Modifikation der Folienrückseite erreicht.

In der EP 1702955 wird eine PSA-Komposition beschrieben, die u.a. auch Alkyl(meth)acrylate enthält. Diese weisen aber keine langkettigen Alkylreste auf, den die Länge der Alkohole ist auf 4 bis 6 Kohlenstoffatome beschränkt.
Für ein besonders günstiges Verhalten dieser Klebemasse auf der Haut ist des weiteren nicht der Acrylester-Anteil verantwortlich, sondern dieses wird erreicht durch einen zusätzlichen Anteil eines Weichmachers. Im Übrigen ist das Ziel der Klebemasse der EP 1702955 ein hartes Ablösen von der Haut.

Auch in der EP 507878 werden PSAs mit kurzkettigen Acryl- oder Methacrylsäureestern, die Kettenlänge liegt hier zwischen 4 und 10 C-Atomen, beschrieben.
Auch hierin wird die Acrylat-Masse nicht als alleinige Klebemasse eingesetzt, sie dient lediglich als "continuous matrix" in einem Zweiphasensystem, das u.a. auch noch ein Hydrokolloid enthält. Dabei hat der Acrylat-Anteil die Aufgabe, das Kleben des Hydrokolloids auf der Haut zu verstärken.

In der EP 1021469 wird ein Trennmittel beschreiben, dessen Ziel die Verhinderung einer unabsichtlichen Verklebung darstellt. Hierin wird u.a. Stearylacrylat genannt, dessen trennwirksame Eigenschaften bekannt sind.
Das zu lösende Problem in der EP 1021469 ist ähnlich dem in der DE 1719167, d.h. bei abzurollenden Klebebändern gilt es ein Umspulen der Klebemasse zu verhindern, um so die Klebemasse des Produktes auf der "richtigen" Seite zu belassen. Naturgemäß wird ein - entsprechendes Trennmittel auf die nicht-klebende Seite eines Trägermaterials beschichtet, die eigentliche Klebemasse wird also durch den Träger vom Trennmittel getrennt.

Klebemassen zur Fixierung von Wundversorgungsprodukten auf der Haut müssen zwei wichtige Funktionen erfüllen, nämlich eine sichere Verklebung des Wundversorgungsproduktes mit der Haut sowie am Ende der Anwendungszeit eine leichte und insbesondere schmerzlose Entfernung von der Haut gewährleisten.

Diese auch bei zahlreichen Verbraucherbefragungen immer wieder erfahrene wichtigste Eigenschaft eines Pflasters ist bislang allerdings nur unter Kompromissen erreichbar, denn die beiden Eigenschaften "sicheres Kleben" und "sanftes Entkleben" sind nur sehr schwer miteinander vereinbar. Ein sicheres Kleben erfordert eher hohe, ein sanftes eher niedrige Klebkräfte. Ersteres könnte zu schmerzhaften Pflasterentfemungen, letzteres zu schmerzlosen Pflasterentfernungen führen. Ein objektives Kriterium zur Differenzierung einer Pflasterentfemung ist beispielsweise die Anzahl der Corneozyten, die beim Abreißen eines Pflasters von der obersten Hautschicht mitgerissen werden und die dann auf der Klebemasse des entfernten Pflasters bestimmbar sind (P. J. Dykes et al., J. Wound Care 10, 7-10 [2001]).

Neben diesen beiden funktionalen Eigenschaften sollten Klebemassen auch so beschaffen sein, dass sie im optimalen Fall keinerlei irritierende oder allergisierende Potentiale aufweisen. Hautverträglichkeit ist damit ein weiterer Aspekt, unter dem Klebemassen gesehen werden müssen. So können beispielsweise nach der Polymerisation der Ausgangssubstanzen zur fertigen Klebemasse noch geringe Mengen an verbleibenden Restmonomeren negative Reaktionen auf der Haut hervorrufen.

Wünschenswert wäre es demnach, eine Klebemasse bereit zu stellen, die eine ausreichende Klebkraft auf der Haut gewährleistet, ein schmerzlose Entklebung sichert und gleichzeitig hautverträglich ist. Neben den verfügbaren Klebmassen eine weitere verbesserte Alternative zur Verfügung zu stellen ist demnach ein erfindungsgemäßes Ziel.

US 4737410 offenbart, dass reine Polyacrylate relativ soft seien, d.h. zu niedrige Scherkräfte besitzen, und beschreibt deshalb entsprechend verstärkte, nicht reine Polymere, die zu einem Teil "A" aus Acrylaten bestehen.

Auch in der EP 554106 A1 wird eine PSA-Komposition beschrieben, die u.a. auch Alkyl(meth)acrylate enthalten kann. Für das gewünschte Klebeverhalten wird jedoch zwingend ein Copolymerisat mit einer Komponente "B" und ggfs. noch einer weiteren Klebkomponenten "C" gebildet.

Die US 2003221776 A1 beschreibt PSAs mit langkettigen (bis C30) Acryl- oder Methacrylsäureestern, speziell für eine Verklebung von Furnieren.
Die offenbarten Acrylat-Monomere sind nicht die alleinigen Klebkomponenten in der finalen Klebemasse. Zudem zeigen die PSAs keinerlei Anzeichen auf eine sanfte Entklebung, wie sie für menschliche Haut erforderlich ist, sondern ganz im Gegenteil eine harte Verklebung von Furnieren.

In der WO 91/16387 A1 werden PSAs beschrieben, die aus Monomeren aufgebaut sind, die ihrerseits nach ganz bestimmten Methoden copolymerisiert werden können. Damit handelt es sich auch in WO 91/16387 A1 nicht um ein reines Polyacrylat.

US 4554324 offenbart zwar monomere Acrylate mit bis zu 14 Kohlenstoffatomen, aber wiederum nur gemeinsam mit einem anderen Nicht- Acrylsäureester-Monomer "C", das als essentiell bezeichnet wird und letztlich dann auch zu einer nicht als reines Polycrylat zu bezeichnenden Klebemasse führt.

Die Erfindung beschreibt reine Acrylatklebemasse umfassend mindestens einen mit Acrylsäure und/oder substituierter Acrylsäure veresterten Alkohol, gewählt aus der Gruppe der langkettigen, mit einer C-Anzahl von mehr als 12, aliphatischen Alkohole, Fettalkohole, Wachsalkohole, Wollwachsalkohole und/oder Sterole.

Der direkte Kontakt zwischen Haut und Klebemasse erfolgt durch adhäsive molekulare Wechselwirkungen in der sogenannten Adhäsionszone. Es wurde festgestellt, dass zu einer ansprechenden Ausbildung dieser adhäsiven Grenzschicht eine gute Benetzung der Hautoberfläche durch die Klebemasse von Vorteil ist.
Der Grad der Benetzung wird dabei im wesentlich durch die Oberflächenspannungen von Haut und Klebemasse bestimmt.
Es ist somit die Benetzung ein erfindungsgemäßes Kriterium für die Qualität der Adhäsion zwischen Klebemasse und Haut.
Es zeigte sich, dass für eine gute Benetzung gilt, dass die Oberflächenspannung der Klebemasse niedriger sein sollte als die sogenannte kritische Oberflächenspannung des Substrates (D. Satas in "Handbook of PSA Technology" (D. Satas ed.), 3rd edition, 52-56 [1999]).
Bei der kritischen Oberflächenspannung handelt es sich um einen normierten Wert der Oberflächenspannung, um unterschiedliche Benetzungsmittel und Substrate besser miteinander vergleichen zu können.
Daten zur kritischen Oberflächenspannung menschlicher Haut liegen dabei um 26 dyn/cm (M. E. Ginn et al., J. Colloid Interface Sci. 26, 146-151 [1968], H. Schott, J. Pharm. Sci. 60, 1893-1895 [1971], und A. Rosenberg et al., J. Pharm. Sci. 62, 920-922 [1973]).
Im Vergleich dazu weisen gängige in Klebemassen eingesetzte Polymere höhere Werte auf, z.B. Polyurethane 29 dyn/cm, Polyacrylate zwischen 28 dyn/cm und 33 dyn/cm und Polystyrole 33 dyn/cm bis 35 dyn/cm.
Allein Silikonklebemassen liegen mit 22 dyn/cm niedriger als die zuvor genannten Klebemassen (D. Satas in "Handbook of PSA Technology" (D. Satas ed.), 3rd edition, 882-889 [1999]).
Erfindungsgemäß ist nun ein Zusammenhang zwischen den Werten der jeweiligen kritischen Oberflächenenergie einer Klebernasse und der Verträglichkeit/Güte ihrer resultierenden Verklebung aufgestellt worden.

Vergleichende Untersuchungen der kritischen Oberflächenspannung menschlicher Haut mit der Oberflächenspannung von humanem Sebum zeigen mit 24 dyn/cm identische Werte, eine Entfernung des Sebums von der Haut durch Waschen oder Entfetten senkt deren Oberflächenspannung, eine Behandlung mit einer pflegenden Wasser-in-Öl Creme erhöht sie dagegen (A. EI-Khyat et al., Skin Res. Technol. 2, 91-96 [1996]). Damit ist gezeigt, dass die Hautoberfläche zwar primär hydrophob ist, durch ihre Lipidschicht, speziell durch das Sebum, aber paradoxerweise polarer und besser benetzbar wird.

Dieses Phänomen einer besseren Benetzbarkeit der Hautoberfläche in Gegenwart von Hautlipiden wurde bislang bei der Entwicklung von Klebemassen nicht berücksichtigt.

Erfindungsgemäß wurde daher einer Acrylatklebemasse durch die Verwendung von Strukturelementen, die dem Sebum vergleichbar sind, eine zusätzliche hautlipidähnliche Eigenschaft und damit auch eine niedrigere Oberflächenspannung als eine Acrylatklebemasse nach dem heutigen Stand der Technik zugewiesen.

Als Strukturelemente, die dem Sebum vergleichbar sind, haben sich Acrylsäureester und/oder substituierte Acrylsäureester von Alkoholen, insbesondere den langkettigen aliphatischen Alkoholen, Fettalkoholen, Wachsalkoholen, Wollwachsalkoholen und/oder Sterolen gezeigt.
Langkettige Alkohole besitzen erfindungsgemäß eine C-Anzahl von mehr als 12.

Dadurch wird eine verbesserte Benetzbarkeit der Hautoberfläche mit der erfindungsgemäßen Klebemasse erreicht. Die Oberflächenspannung erfindungsgemäß modifizierter Acrylat-Klebemassen sinkt - je nach Höhe des Anteils hautlipidähnlicher Zusätze - von ca. 30 dyn/cm in Richtung auf den Wert der Haut (24 - 26 dyn/cm).
Durch den Zusatz von mindestens einem mit Acrylsäure oder substituierter Acrylsäure veresterten Alkohol wird das Klebeverhalten einer solchen modifizierten Acrylat-Klebemasse verändert. Es ist damit erstmalig möglich, Acrylatklebemasse bereit zu stellen, die ein sanftes Klebeverhalten zeigen, das bislang nur für Siliconklebemassen bekannt ist.

Es hat sich überraschend gezeigt, dass durch die Inkorporation von Acrylsäureestern und/oder substituierten Acrylsäureestern von Alkoholen, insbesondere den langkettigen aliphatischen Alkoholen, Fettalkoholen, Wachsalkoholen, Wollwachsalkoholen und/oder Sterolen, in Acrylatklebemassen diese dann eine erniedrigte Oberflächenspannung ergeben. Die dann der Haut ähnlicheren Oberflächenspannungswerte der Klebemasse ermöglichen einerseits eine gute Verklebung und gleichzeitig aber auch eine sanftere, weniger schmerzhafte Entklebung beim Wiederablösen.

Der Vorteil ist unverkennbar: der Anwender kann nach längerem Tragen die mit der erfindunsgemäßen Klebmassen versehen Hautaufkleber, wie Pflaster, schmerzfrei ablösen.

Langkettige aliphatische Alkohole umfassen erfindungsgemäß Alkohole mit einer C-Anzahl von mehr als 12.

Fettalkohole bezeichnen erfindungsgemäß die durch Reduktion der z.B. aus Triglyceriden oder Fettsäuremethylestern herstellbaren Fettsäuren erhältlichen linearen, gesättigten oder ungesättigten primären Alkohole (1-Alkanole) mit bevorzugt 6-22 Kohlenstoff-Atomen, bevorzugt 13-22 C-atome. Dies sind insbesondere die in Tabelle 1 aufgelisteten Alkohole.

**Tabelle 1: Fettalkohole.**

| Alkohol | Summen-Formel |
|---|---|
| Hexan-1-ol (Capronalkohol) | C₆H₁₄O |
| 1-Heptanol (Önanthalkohol) | C₇H₁₆O |
| 1-Octanol (Caprylalkohol) | C₈H₁₈O |
| 1-Nonanol (Pelargonalkohol ) | C₉H₂₀O |
| 1-Decanol (CaprinalKohol) 1-Undecanol | C₁₀H₂₂O C₁₁H₂₄O |
| Undeo-10-en-1-ol | C₁₁H₂₂O |
| 1-Dodecanol (Laurylalkohol) | C₁₂H₂₆O |
| 1-Tridecanol | C₁₃H₂₈O |
| 1-Tetradecanol (Myristylalkohol) | C₁₄H₃₀O |
| 1-Pentadecanol | C₁₅H₃₂O |
| (Cetylalkohol) | |
| 1-Heptadecanol | C₁₇H₃₆O |
| 1-Octadecanol (Stearylalkohol) | C₁₈H₃₈O |
| 9-cis-Octadecen-1-ol (Oleylalkohol) | C₁₈H₃₆O |
| 9-trans-Octadecen-1-ol (Erucylalkohol) | C₁₈H₃₆O |
| 9-cis-Octadecen-1,12-diol (Ricinolalkohol) | C₁₈H₃₆O₂ |
| all-cis-9,12-Octadecadien-1-ol (Linoleylalkohol) | C₁₈H₃₄O |
| all-cis-9,12,15-Octadecatrien-1-ol (Linolenylalkohol) | C₁₈H₃₂O |
| 1-Nonadecanol | C₁₉H₄₀O |
| 1-Eicosanol (Arachidylalkohol) | C₂₀H₄₂O |
| 9-cis-Eicosen-1-ol (Gadoleylalkohol) | C₂₀H₄₀O |
| 5,8,11,14-Eicosatetraen-1-ol | C₂₀H₃₄O |
| 1-Heneicosanol | C₂₁H₄₄O |
| 1-Docosanol (Behenylalkohol) | C₂₂H₄₆O |
| 13-cis-Docosen-1-ol (Erucylalkohol) | C₂₂H₄₄O |
| 13-trans-Docosen-1-ol (Brassidylalkohol) | C₂₂H₄₄O |

Erfindungsgemäß zählen auch die höhermolekularen Alkohole, wie z.B. Lignocerylalkohol (C₂₄H₅₀O), Cerylalkohol (C₂₆H₅₄O), oder Myricylalkohol (C₃₀H₆₂O) als sogenannte Wachsalkohole zu den bevorzugt auszuwählenden und mit Acrylsäure und/oder substituierter Acrylsäure zu veresterden Alkoholen.
Wachsalkohole umfassen erfindungsgemäß höhermolekulare meist wasserunlösliche Fettalkohole mit ca. 24-36 Kohlenstoff-Atomen, die in Form von Wachsestern höhermolekularer Fettsäuren (Wachssäuren) Hauptbestandteil vieler natürlicher Wachse sind. Bevorzugte Beispiele der Wachsalkohole sind Lignocerylalkohol, Cerylalkohol, Myricylalkohol oder Melissylalkohol.

Bevorzugte Alkohole im Sinne der Erfindung sind insbesondere Wollwachsalkohole. Wollwachsalkohole umfassen die unverseifbare Fraktion des Wollwachses. Die Wollwachsalkohole nehmen selbst kein Wasser auf, verleihen aber z.B. Vaseline oder anderen geeigneten Kohlenwasserstoffen ein hohes Wasseraufnahmevermögen. Die Wollwachsalkohole werden daher vornehmlich zur Herstellung von Salbengrundlagen, aus denen Wasser-in-Öl-Emulsionen hergestellt werden, und als Emulgatoren verwendet.
In der Kosmetik werden bekannterweiser Wollwachsalkohole als Emulgatoren oder Hautpflegemittel eingesetzt.
Diese Wollwachsalkohole sind den Hautfettalkoholen strukturell und hinsichtlich ihrer Polaritäten ähnlich (N. Nicolaides, Science 186, 19-26 [1974], und K. Motiuk, J. Am. Oil Chem. Soc. 56, 651-658 [1979]).
Ihre Verwendung in Klebemassen ist allerdings bislang nicht bekannt, allein die mit Wollwachssäuren veresterte Form der Wollwachsalkohole, das Wollwachs selbst, wird als Weichmacher in Kautschukmassen eingesetzt, DE 19824071 A1.
Unter Wollwachs werden erfindungsgemäß auch Triterpenoid- und Steroid-Alkohole verstanden.

In gleicher Weise sind die Sterole bevorzugt zu wählende Alkohole. Dazu zählen insbesondere die von Cholesterol abgeleitete Gruppe natürlich vorkommender Steroide, die eine 3 β-Hydroxy-Gruppe und eine 17 β-ständige aliphatische, in der Regel aus 8-10 Kohlenstoff-Atomen bestehende Seitenkette aufweisen. Erfindungsgemäß sind die folgenden Sterole bevorzugt 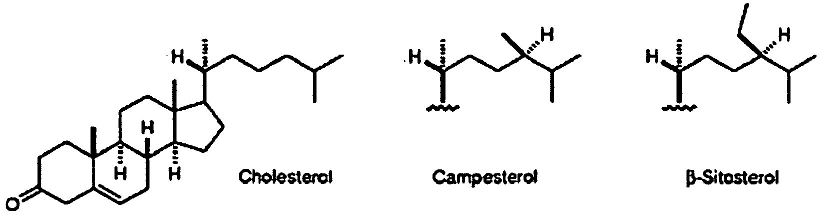 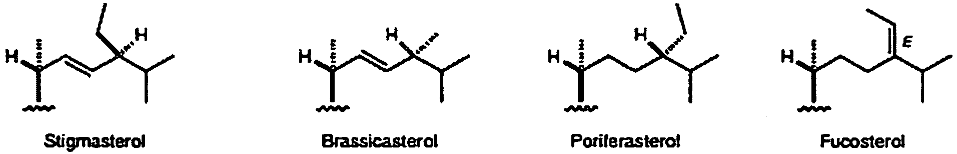 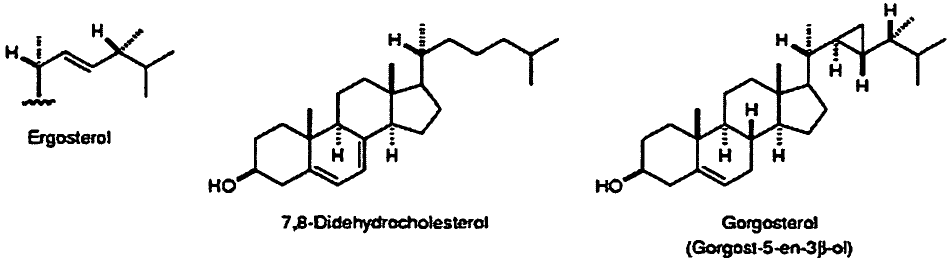 sowie Lanosterol

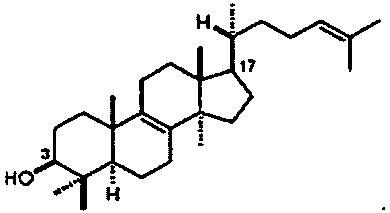

Besonders bevorzugte Sterole sind Cholesterol, Cholecalciferol, Lanosterol, Ergosterol und/oder Campesterol.

Aufgrund der freien Hydroxylgruppen in den Fettalkoholen, Wollwachsalkoholen oder auch in den analogen langkettigen aliphatischen Alkoholen oder Sterolen können diese beispielsweise in entsprechende Ester der Acrylsäure oder substituierter Acrylsäuren, wie z.B. der Methacrylsäure, umgeformt werden.

Im Falle langkettiger aliphatischer Fettalkohole gelingt dieses durch einfache Umesterung von Methyl- oder Ethylestem der Acrylsäure oder der Methacrylsäure.
So erhältliches Stearylacrylat ist beispielsweise als ein hochinteressantes Monomer für die Herstellung von Formgedächtnispolymeren beschrieben (A. Lendlein und S. Kelch, Angew. Chem. 114, 2138-2162 [2002]). Auch die Ester der Wollwachssterole sind bekannt. Cholesterylacrylat wird zum Beispiel durch direkte Umsetzung des Sterols mit Acryloylchlorid gewonnen (US 6,147,068).
Anwendungsgebiete für Cholesterylacrylat sind beschrieben für die sog. "Molecular Imprinting Technology" (N. Zhong et al., Tetrahedron Lett. 42, 1839-1841 [2001]) oder für die Herstellung von Flüssigkristallen (P. J. Shannon, US Patent 4,614,619 [1986], und D. Filip et al., Mat. Res. Bull. 36, 1455-1461 [2001]).

Besonders bevorzugte Alkohole sind Palmitylalkohol, Stearylalkohol, Oleylalkohol, Cholesterol, Lanosterol, Dihydrolanosterol und/oder Wollwachsalkoholgemische wie z.B Eucerit^{®}, da diese als Hauptkomponenten im Sebum und auch im Wollwachs maßgeblich für die Senkung der Oberflächenspannung verantwortlich sind.

Diese Alkohole werden erfindungsgemäß als einzelne Acrylsäure-, Methacrylsäure- oder Cyanacrylsäureester oder in Mischungen in die Acrylatklebemasse eingesetzt.

Die erfindungsgemäße Klebemasse umfasst demnach bevorzugt Acrylatester der Wollwachsalkohole und/oder -sterole, insbesondere Stearyl-, Palmityl-, Cholesteryl-, Lanosteryl- und/oder Dihydrolanosterylacrylat, -methacrylat und/oder -cyanacrylat und/oder deren Mischungen.

Erfindungsgemäß wird ein sanftes und hautverträgliches Kleben durch den Einsatz solcher Acrylsäure-, Methacrylsäure- oder Cyanacrylsäureester erreicht, die als alkoholische Komponente Alkohole enthalten, die im humanen Sebum oder im Wollwachs vorkommen.

Erfindungsgemäß sind unter Acrylatklebemasse alle Klebmassen auf Basis von AcrylMonomeren, insbesondere von Acryl- und Methacrylsäureestern sowie Cyanacrylsäureester zu verstehen. Sie werden erfindungsgemäß monomer als Reaktionsklebstoffe eingesetzt, die während des Verklebungsprozesses polymerisieren, oder bereits als Polymer, Polyacrylate, Polymethacrylate oder Polycyanacrylate in Form von Lösungen- bzw. Dispersionsklebstoffen eingesetzt.

Polymethacrylate oder Polycyanacrylate in Form von Lösungen- bzw. Dispersionsklebstoffen eingesetzt.

Vorteilhafte Polyacrylate sind beispielsweise Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt werden. Insbesondere zeichnen sich das oder die vorteilhaften Acrylat-Alkylacrylat-Copolymere durch die folgende Struktur aus:

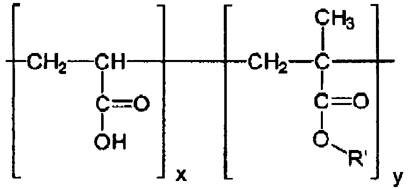

Darin stellen R' einen langkettigen Alkylrest und x und y Zahlen dar, welche den jeweiligen stöchiometrischen Anteil der jeweiligen Comonomere symbolisieren.

Ferner vorteilhaft sind Copolymere aus C₁₀₋₃₀-Alkylacrylaten und einem oder mehreren Monomeren der Acrylsäure, der Methacrylsäure oder deren Ester, die kreuzvemetzt sind mit einem Allylether der Saccharose oder einem Allylether des Pentaerythrit.

Weitere Bestandteile der Matrix können Lösungsvermittler, z.B. Polyethylenglycole (Lutrol E400, E600 der Firma BASF) in einer Menge von 0 - 50 Gew.%, bevorzugt 0 - 30 Gew.%. Neutralisationsmittel, z.B. Tromethamol, Triethanolamin und/oder Dexpanthenol, in einer Menge 0 - 30 Gew.%, bevorzugt 0 - 15 Gew.%, Füllstoff(e), z.B. Kieselsäure, mikronisierte Cellulose und/oder Gelatine, in einer Menge von 0 - 30 Gew.%, bevorzugt 3 - 15 Gew.%, und natürlich Wirkstoff(e), z.B. Menthol, Jojobaöl, Ibuprofen, Benzylnicotinat und/oder Capsaicin, in einer Menge von 0-35 Gew.%, bevorzugt 0 - 15 Gew.%, sein.

Die erfindungsgemäße Klebmasse ist eine reine Acrylat-Klebemasse, deren kritische Oberflächenspannung aufgrund der spezifischen Eigenschaft langkettiger O-Alkylreste in der Größenordnung der Oberflachenspannung der menschlichen Haut einstellbar ist. Andere Klebmassen mit Acrylatanteilen, wie Acrylkautschuk stellen keine erfindungsgemäße reine Acrylatmasse dar.

Die Herstellung der erfindungsgemäßen Acrylatklebemasse erfolgt wie aus dem Stand der Technik bekannt, ggf. lösemittelfrei, vorzugsweise bei Raumtemperatur, in handelsüblichen Knetern oder geeigneten Extrudern. Am Pflasteraufbau würde sich gegenüber dem bekannten Aufbau keine Änderungen ergeben, was vorteilhaft eine Herstellverfahrensumstellung vermeidet.

Übliche Acrylatklebemassen für Pflaster enthalten als vom Molekulargewicht her gesehen größtest Monomer 2-Ethyl-hexylacrylat (US 5,876,855). Höhermolekulare aliphatische Acrylat-Monomere oder Stearyl-Gruppen enthaltende Acrylat-Monomere sind für derartige Pflastermassen nicht beschrieben. Allerdings findet sich bei der Herstellung von Copolymerisaten als Trennmitteldispersionen zur klebstoffabweisenden Beschichtung auf flächigem Material ein Hinweis darauf, dass Stearylacrylat und/oder -methacrylat im Copolymerisat zur Erhöhung der Trennwirkung beitragen, da sie zu den trennwirksamen Monomeren gehören (DE 19926169).

Mit Acrylsäure oder substituierten Acrylsäuren veresterte Alkohole, Wollwachsalkohole oder Hautfettalkohole wie z.B. Palmitylalkohol, Stearylalkohol, Oleylalkohol, Cholesterol, Lanosterol, Dihydrolanosterol oder Kombinationen davon oder mit Acrylsäure oder substituierten Acrylsäuren veresterte Wollwachsalkoholgemische wie z.B Eucerit^{®} weisen nach Polymerisation dem menschlichen Sebum ähnliche Oberflächenspannungen auf.
Die Acrylatester können deshalb in Anteilen von bevorzugt mindestens 5 Gew.% und vorteilhaft maximal 40 Gew.%, bezogen auf die Gesamtmasse der Klebemasse, in gängigen Acrylatklebemassen eigesetzt werden. Sie führen dann zu einer Senkung der Oberflächenspannung der Acrylatklebemasse ohne die Zusätze und verschieben diese erfindungsgemäß in die Größenordnung der menschlichen Haut.
Auf diese Weise gelingt es, besonders sanft klebende Acrylatklebemassen zu erhalten. Da außerdem etwaige nicht vollständig auspolymerisierte Restmonomere Hautfett-ähnliche oder sogar -identische Strukturen aufweisen, wird auch das hautreizende oder allergisierende Potential weiter minimiert.

Insgesamt führt der Einsatz der erfindungsgemäßen Kombination der Substanzen zu Acrylatklebemassen, die ein sanftes Kleben gewährleisten und zusätzlich besonders hautverträglich sind.
Dies wurde eindrucksvoll in Probandentests ermittelt. Es wurden dabei erfindunsgemäße Pflaster im Vergleich zu handelsüblichen Pflaster auf die Haut der Probanden geklebt und nach verschiedenen Zeiten deren Klebkraft und Ablöseverhalten subjektiv beurteilt.

Bevorzugte Acrylatklebemassezusammensetzungen weisen vorteilhaft einen Anteil von 15 Gew.% Cholesterylacrylat und 10 Gew. % Stearylacrylat auf.

Die Verwendung von mindestens einem mit Acrylsäure und/oder substituierter Acrylsäure veresterten Alkohol, gewählt aus der Gruppe der langkettigen, mit einer Anzahl von 12, aliphatischen Alkohole, Fettalkohole, Wachsalkohole, Wollwachsalkohole und/oder Sterole, führt zu einer Verbesserung der Haftung, Enthaftung und/oder Hautverträglichkeit der Acrylatklebemassen.

Bevorzugt lassen sich mit den erfindungsgemäßen Klebemassen Pflaster aber auch selbstklebende Hautauflagen, patches, wie z.B. Anti-Cellulite patches oder pads herstellen.

Pflaster oder Patches mit erfindungsgemäßer Klebemasse sind insbesondere im Baby- und Kinderbereich anzuwenden sowie bei Verletzungen an besonders sensiblen Hautpartien wie z.B. im Gesicht. Auch das Thema Narbenpflaster kann mit den erfindungsgemäßen Klebemassen besser bedient werden, weil dort für eine längere Zeit immer wieder auf derselben Hautstelle geklebt werden muss, wodurch die sanften entklebenden und hautverträglichen erfindungsgemäßen Klebemassen bevorzugt sind.

Weiterhin vorteilhaft sind Klebemassen, bei denen der Alkoholanteil, sozusagen als Emulgatorenanteil, in einem Bereich zwischen 1 und 10 Gew.% liegt, wobei bei gleichzeitiger Verwendung von Estern langkettiger Fettalkohole und Sterolestern deren Verhältnis ca. 3:7 beträgt.

In drei Beispielen sind Standard-Acrylatklebemassen mit Anteilen von
a) 5 Gew.% Cholesterylacrylat
b) 10 Gew.%% Stearylacrylat
c) 2 Gew.% Stearylacrylat + 4,5 Gew.%% Cholesterylacrylat versehen worden.

Alle drei Klebemassen zeichnen sich durch ein sicheres Kleben, schmerzfreis Entkleben und einer optimierten Hautverträglichkeit aus.

Die beschriebenen modifizierten Klebemassen können insbesondere für Pflaster verwendet werden, die auf sensibler Haut benutzt werden. Entsprechend ausgestattete Pflaster erlauben bei Kindern und bei älteren Menschen eine sanfte Verklebung mit einer anschließend schmerzlosen Entfernung.

Des weiteren ermöglichen sie bei einer längerfristigen Anwendung von Pflastern auf ein und demselben Hautareal eine geringere ständige Ablösung oberer Hautschichten beim Pflasterwechsel mit entsprechend reduzierten Hautirritationen.
Darüber hinaus erlauben sie bei Pflastern, die kurzzeitig abgehoben und dann erneut aufgeklebt werden sollen, eine verbesserte Repositionierung.

## Patentansprüche

1. Reine Acrylatklebemasse umfassend mindestens einen mit Acrylsäure und/oder substituierter Acrylsäure veresterten Alkohole, gewählt aus der Gruppe der langkettigen, mit einer C-Anzahl von mehr als 12, aliphatischen Alkohole, Fettalkohole, Wachsalkohole, Wollwachsalkohole und/oder Sterole.

2. Klebemasse nach Anspruch 1, **dadurch gekennzeichnet, dass** als Alkohole Wollwachsalkohole, Hautfettalkohole und/oder deren Gemische gewählt werden.

3. Klebemasse nach Anspruch 1 oder 2, **dadurch gekennzeichnet, dass** als Alkohole Palmitylalkohol, Stearylalkohol, Oleylalkohol, Cholesterol, Lanosterol, Dihydrolanosterol und/oder Wollwachsalkoholgemische gewählt werden.

4. Klebemasse nach einem der vorstehenden Ansprüche auf Basis von Acryl-, Methacryl-, und/oder Cyanacrylsäureester.

5. Klebemasse nach einem der vorstehenden Ansprüche, **dadurch gekennzeichnet, dass** die Ester zu einem Anteil von mindestens 5 Gew.%, bezogen auf die Gesamtmasse der Klebemassen, enthalten sind.

6. Klebemasse nach einem der vorstehenden Ansprüche, **dadurch gekennzeichnet, dass** die Ester zu einem Anteil von maximal 40 Gew.%, bezogen auf die Gesamtmasse der Klebemassen, enthalten sind.

7. Verwendung der Klebemasse nach einem der vorstehenden Ansprüche zur Herstellung von Pflaster, selbstklebenden Hautauflagen, patches und/oder Pads.

8. Verwendung von mindestens einem mit Acrylsäure und/oder substituierter Acrylsäure veresterten Alkohol, gewählt aus der Gruppe der langkettigen, mit einer Anzahl von mehr als 12, aliphatischen Alkoholen, Fettalkohole, Wachsalkohole, Wollwachsalkohole und/oder Sterole, zur Verbesserung der Haftung, Enthaftung und/oder Hautverträglichkeit von Acrylatklebemassen.

## Claims

1. Pure acrylate adhesive mass comprising at least one alcohol esterified with acrylic acid and/or substituted acrylic acid, selected from the group of long-chain aliphatic alcohols, fatty alcohols, wax alcohols, wool wax alcohols and/or sterols with a carbon number of more than 12.

2. Adhesive mass according to Claim 1, **characterized in that** wool wax alcohols, skin fatty alcohols and/or mixtures thereof are selected as alcohols.

3. Adhesive mass according to Claim 1 or 2, **characterized in that** palmityl alcohol, stearyl alcohol, oleyl alcohol, cholesterol, lanosterol, dihydrolanosterol and/or wool wax alcohol mixtures are selected as alcohols.

4. Adhesive mass according to one of the preceding claims based on acrylic, methacrylic and/or cyano-acrylic acid esters.

5. Adhesive mass according to one of the preceding claims, **characterized in that** the esters are present to a fraction of at least 5% by weight, based on the total mass of the adhesive masses.

6. Adhesive mass according to one of the preceding claims, **characterized in that** the esters are present to a fraction of at most 40% by weight, based on the total mass of the adhesive masses.

7. Use of the adhesive mass according to one of the preceding claims for producing plasters, self-adhesive skin coverings, patches and/or pads.

8. Use of at least one alcohol esterified with acrylic acid and/or substituted acrylic acid, selected from the group of long-chain aliphatic alcohols, fatty alcohols, wax alcohols, wool wax alcohols and/or sterols with a carbon number of more than 12 for improving the adhesion, release and/or skin compatibility of acrylate adhesive masses.

## Revendications

1. Matière adhésive acrylate pure comprenant au moins un alcool estérifié avec de l'acide acrylique et/ou de l'acide acrylique substitué, choisi dans le groupe des alcools aliphatiques à chaîne longue ayant un nombre de C supérieur à 12, des alcools gras, des alcools de cire, des alcools de lanoline et/ou des stérols.

2. Matière adhésive selon la revendication 1, **caractérisée en ce que** des alcools de lanoline, des alcools gras de la peau et/ou leurs mélanges sont choisis en tant qu'alcools.

3. Matière adhésive selon la revendication 1 ou 2, **caractérisée en ce que** l'alcool palmitylique, l'alcool stéarylique, l'alcool oléylique, le cholestérol, le lanostérol, le dihydrolanostérol et/ou des mélanges d'alcools de lanoline sont choisis en tant qu'alcools.

4. Matière adhésive selon l'une quelconque des revendications précédentes, à base d'esters d'acide acrylique, méthacrylique et/ou cyanoacrylique.

5. Matière adhésive selon l'une quelconque des revendications précédentes, **caractérisée en ce que** les esters sont contenus en une proportion d'au moins 5 % en poids, par rapport à la masse totale des matières adhésives.

6. Matière adhésive selon l'une quelconque des revendications précédentes, **caractérisée en ce que** les esters sont contenus en une proportion d'au plus 40 % en poids, par rapport à la masse totale des matières adhésives.

7. Utilisation de la matière adhésive selon l'une quelconque des revendications précédentes pour la fabrication de pansements, de compresses cutanées autoadhésives, de timbres cutanés et/ou de tampons.

8. Utilisation d'au moins un alcool estérifié avec de l'acide acrylique et/ou de l'acide acrylique substitué, choisi dans le groupe des alcools aliphatiques à chaîne longue ayant un nombre de C supérieur à 12, des alcools gras, des alcools de cire, des alcools de lanoline et/ou des stérols, pour améliorer l'adhésion, le décollement et/ou la compatibilité avec la peau de matières adhésives acrylates.
